# EUROPEAN PATENT SPECIFICATION

(11) **EP 2 593 527 B1**
(45) Date of publication and mention of the grant of the patent: **02.04.2014**
(21) Application number: 11744082.6
(22) Date of filing: 08.07.2011
(51) Int. Cl.: H01J 61/44, C09K 11/80

(54) **UV-A OR UV-B-EMITTING DISCHARGE LAMP**
UV-A ODER UV-B EMITTIERENDE ENTLADUNGSLAMPE
LAMPE À DÉCHARGE ÉMETTANT DES UV-A OU DES UV-B

(30) Priority: 13.07.2010 EP 10169377
(43) Date of publication of application: 22.05.2013
(73) Proprietor: Koninklijke Philips N.V., 5656 AE Eindhoven (NL); Philips Intellectual Property & Standards GmbH, 20099 Hamburg (DE)
(72) Inventor: JUESTEL, Thomas, NL-5656 AE Eindhoven (NL); GREUEL, Georg, NL-5656 AE Eindhoven (NL)
(74) Representative: van Eeuwijk, Alexander Henricus Waltherus
(86) International application number: PCT/IB2011/053045
(87) International publication number: WO 2012/007885

(56) References cited:
- EP-A1- 1 816 241
- WO-A1-2010/097731
- WO-A2-2005/124825
- FR-A1- 2 351 498
- JP-A- 2008 231 334
- CICILLINI S A ET AL: "Luminescent and morphological studies of Tm-doped Lu3Al5O12 and Y3Al5O12 fine powders for scintillator detector application", JOURNAL OF ALLOYS AND COMPOUNDS, ELSEVIER SEQUOIA, LAUSANNE, CH, vol. 374, no. 1-2, 14 July 2004 (2004-07-14), pages 169-172, XP004514921, ISSN: 0925-8388, DOI: 10.1016/J.JALLCOM.2003.11.085
- PATEL D N ET AL: "PHOTON-AVALANCHE UPCONVERSION IN THULIUM-DOPED LUTETIUM ALUMINUM GARNET", APPLIED OPTICS, OPTICAL SOCIETY OF AMERICA, WASHINGTON, DC; US, vol. 38, no. 15, 20 May 1999 (1999-05-20), pages 3271-3274, XP000835183, ISSN: 0003-6935, DOI: 10.1364/AO.38.003271

## Description

### FIELD OF THE INVENTION

The present invention is directed to novel materials for light emitting devices, especially to the field of novel materials for discharge lamps emitting UV radiation.

### BACKGROUND OF THE INVENTION

Fluorescent lamps which comprise an UV emitting phosphor are widely applied for cosmetic and medical purposes. These lamps usually generate UV light by e.g. utilizing an Hg low-pressure discharge and a luminescent screen comprising UV-B or UV-A phosphors or a blend of several UV-A/B phosphors. The most commonly applied phosphors are LaPO₄:Ce, SrAl₁₂O₁₉:Ce,Na, or LaB₃O₆:Bi,Gd as UV-B emitter and (Y,Gd)PO₄:Ce, BaSi₂O₅:Pb, or SrB₄O₇:Eu as UV-A emitter.

However, Hg discharge lamps for disinfection or purification purposes do not use a luminescent screen, since the spectral power distribution of the emission spectrum of a low-or medium-pressure Hg discharge nicely coincides with the GAC and with the absorption edge of molecular Oxygen and water.

The main drawback of the presently applied low- and medium-pressure Hg discharge lamps is their strong dependence on the radiation output from the (water, air) temperature. This is caused by the vapor pressure of liquid Hg, which is a sensitive function of temperature.

Moreover, chemical interaction between the Hg discharge and the glass vessel causes the formation of color centers, which in turn causes an efficiency reduction. Formation of an absorbing layer due to color centers is reduced, once nanoscale particles or phosphor particles are coated on top of the glass surface. Therefore, low- and medium-pressure Hg discharge lamps show strong degradation and must be replaced after about 1000 h operation.

Therefore, there is the need for alternative phosphors, especially for UV-A and/or UV-B lamps, which at least partly overcome the above-mentioned drawbacks and which have a longer lifetime.

### SUMMARY OF THE INVENTION

It is an object of the present invention to provide a discharge lamp which is at least partly able to overcome the above-mentioned drawbacks, and especially to enable building a discharge lamp with good or improved lighting features together with an increased lifetime for a wide range of applications.

This object is achieved by a discharge lamp according to claim 1 of the present invention. Accordingly, a discharge lamp, preferably a low-pressure gas discharge lamp, is provided, wherein said discharge lamp is provided with a discharge vessel comprising a gas filling with a discharge-maintaining composition, wherein at least a part of a wall of the discharge vessel is provided with a luminescent material comprising Lu_{1-x-y-a-b}YₓGd_{y})₃Al_{5z}Ga_{z}O₁₂:RE1ₐ:RE2_{b}, with RE1 being Tm³⁺ or mixtures thereof with Pr³⁺, RE2 being selected out of the group comprising Nd³⁺, Sm³⁺, Eu³⁺, Tb³⁺, Dy³⁺, Ho³⁺, Er³⁺ or mixtures thereof and a is >0 and <0.1, b is ≥0 and ≤0.1, x is ≥0 and ≤1, y is ≥0 and ≤1 (with the proviso that a+b+x+y are ≤1) and z is ≥0 and ≤5.

Surprisingly, it has been found that, for a wide range of applications within the present invention, such a discharge lamp has at least one of the following advantages:
a higher lamp efficacy due to the optimized emission spectrum to the action curve of the application and due to less re-absorption by the phosphor layer,
a higher stability of the UV output and thus a higher operational lifetime of the UV light source
   the ease of application of fast switching cycles and the fast lamp run-up time
an adjustable UV emission spectrum
and a high flexibility of the lamp with respect to its geometry and switching

Preferably the discharge lamp is a Xe, Ne, or Xe/Ne excimer discharge lamp.

According to a preferred embodiment of the present invention, especially if RE1 comprises Pr, then z is ≥0.5 and ≤5, i.e. the luminescent material comprises Praseodymium and Gallium. Surprisingly, the inventors have found out that, as a result thereof, a blue-shift emission of the resulting luminescent material can be observed which leads to luminescent materials which are highly desirable for many applications within the present invention. This effect can be observed for many applications with Tm as well, although for Pr it is somewhat more dominant. However, also embodiments are preferred where RE1 comprises Tm and z is ≥0.5 and ≤5.

According to a preferred embodiment of the present invention z is ≥1 and ≤3.

According to a further preferred embodiment, at least a part of a wall of the discharge vessel is provided with a basic metal oxide, preferably selected out of the group comprising MgO, MgAl₂O_{4,} Al₂O₃, or Ln₂O₃ (Ln = Sc, Y, La, Gd, Lu) or mixtures thereof. This has been shown to be advantageous for many applications within the present invention due to the observed reduction of the decrease in radiation output over the lifetime.

The basic metal oxide is preferably provided as a nanoscale particle material with an average particle size of ≥10 nm and ≤1 µm, preferably ≥100 nm and ≤ 500nm. Preferably, it is provided either together with the luminescent material (e.g. a layer is formed comprising both materials) or as a layer on top of or underneath the luminescent material.

According to a preferred embodiment, at least a part of a wall of the discharge vessel is provided with a further luminescent material selected out of the group comprising (Y₁₋ₓ₋ₐLuₓ)BO₃:Prₐ, (Y₁₋ₓ₋ₐLuₓ)PO₄:Prₐ, (Y₁₋ₓ₋ₐLuₓ)PO₄:Biₐ, La₁₋ₐPO₄:Prₐ, (Y₁₋ₓ₋ₐLuₓ)AlO₃:Prₐ, (Ca₁₋ₓ₋₂ₐSrₓ)₂P₂O₇:PrₐNaₐ, (Ca₁₋ₓ₋₂ₐSrₓ)Al₁₂O₁₉:CeₐNaₐ, or (La₁₋ₓ₋ₐGdₓ)PO₄:Ceₐ with x ≥0 and ≤1 and a is >0 and ≤0.1 (for all materials) or mixtures thereof.

By virtue thereof, for many applications an optimization of the lamp spectrum to the action spectrum of the given application is feasible.

The present invention furthermore relates to the use of (Lu_{1-x-y-a-b}YₓGd_{y})₃Al_{5z}Ga_{z}O₁₂:RE1ₐ:RE2_{b}, with RE1 being Tm³⁺ or mixtures thereof with Pr³⁺, RE2 being selected out of the group comprising Nd³⁺, Sm³⁺, Eu³⁺, Tb³⁺, Dy³⁺, Ho³⁺, Er³⁺ or mixtures thereof and a is >0 and <0.1, b is ≥0 and ≤0.1, x is ≥0 and <1, y is ≥0 and ≤1 (with the proviso that a+b+x+y are ≤1) and z is ≥0 and ≤5 as an activator in UV-A and/or UV-B emitting illumination systems.

A system comprising a discharge lamp as described herein or making use of the phosphor material as described above may be used in one or more of the following applications:
Equipment for medical therapy (e.g. Treatment of skin diseases such as Psoriasis)
Equipment for cosmetic skin treatment (e.g. tanning devices)
Water sterilization and/or purification applications, e.g. by using an additional TiO₂ photocatalyst
Chemical reactors, e.g. for the photochemical synthesis of advanced chemical products, e.g. Vitamin D₃

The aforementioned components, as well as the claimed components and the components to be used in accordance with the invention in the described embodiments, are not subject to any special exceptions with respect to their size, shape, Discharge lamp selection and technical concept, so that the selection criteria known in the pertinent field can be applied without limitations.

### BRIEF DESCRIPTION OF THE DRAWINGS

Additional details, features, characteristics and advantages of the object of the invention are disclosed in the subclaims, the Figures and the following description of the respective Figures and examples, which --in exemplary fashion-- show several embodiments and examples of discharge lamps according to the invention.
Fig. 1 shows a very schematic cross-sectional view of a discharge lamp according to a first embodiment of the present invention.
Fig. 2 shows an emission spectrum of a first luminescent material according to the present invention (Example I).
Fig. 3 shows the excitation spectrum of the material of Fig. 2.
Fig. 4 shows an emission spectrum of a second luminescent material according to the present invention (Example II).
Fig. 5 shows the excitation spectrum of the material of Fig. 4.
Fig. 6 shows an emission spectrum of a second luminescent material according to the present invention (Example III).
Fig. 7 shows the excitation spectrum of the material of Fig. 6.
Fig. 8 shows the emission spectrum of a Xe excimer discharge lamp comprising the materials of Examples I and II.

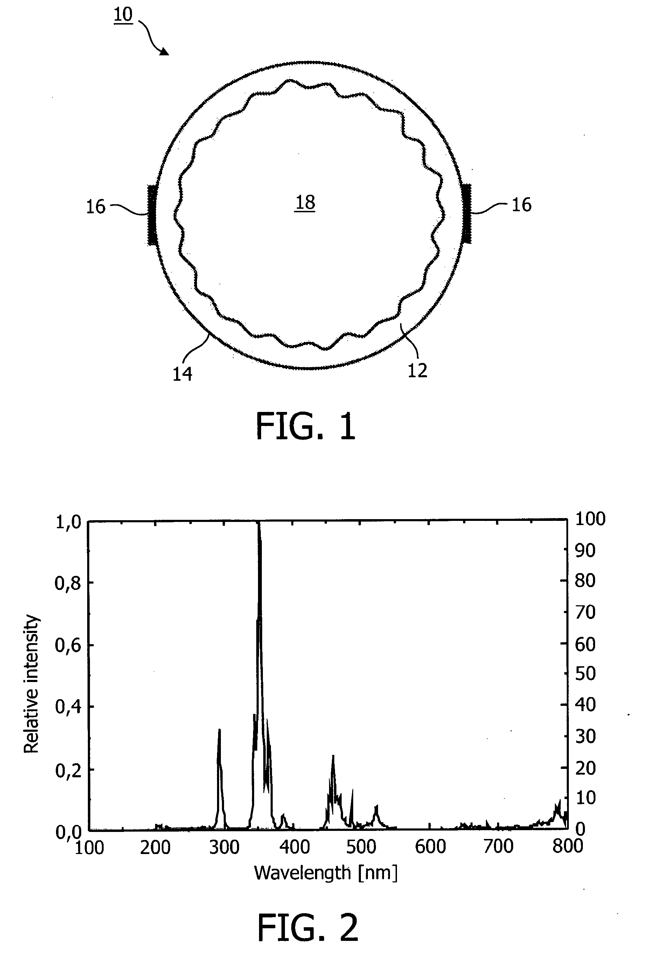

Fig. 1 shows a very schematic cross-sectional view of a discharge lamp according to a first embodiment of the present invention. The discharge lamp 10 (which is principally prior art) comprises a glass tube 14 in which a phosphor 12 is provided. This phosphor comprises the luminescent material of the present invention. Depending on the application also further phosphors and basic metal oxide nanoparticle may be present in this area. Furthermore two electrodes (e.g. made of Al) 18 are provided.

The invention will furthermore be understood by the following Inventive Example which is merely for illustration of the invention only and non- limiting.

### EXAMPLE I

Example I refers to Lu₃Al₅O₁₂:Tm, which was made in the following way:

The starting materials 59.093 g (148.5 mmol) Lu₂O₃, 24.471 g (240 mmol) Al₂O₃ and 1.014 g (3.0 mmol) Tm₂O₃ are dissolved in conc. HNO₃. Then the solvent is removed by evaporation and the remaining powder is fired for 2 h at 600 °C to decompose the nitrates.

Subsequently, the obtained is powderized and 1.680 g (20 mmol) AlF₃ are added as a flux. Afterwards, the powder is annealed for 2 h at 1300 °C in a CO atmosphere, powderized and fired again for 4 h between 1500 and 1700 °C in a CO atmosphere. Finally, the obtained powder cake is crushed and the powder is sieved through a 36 µm sieve.

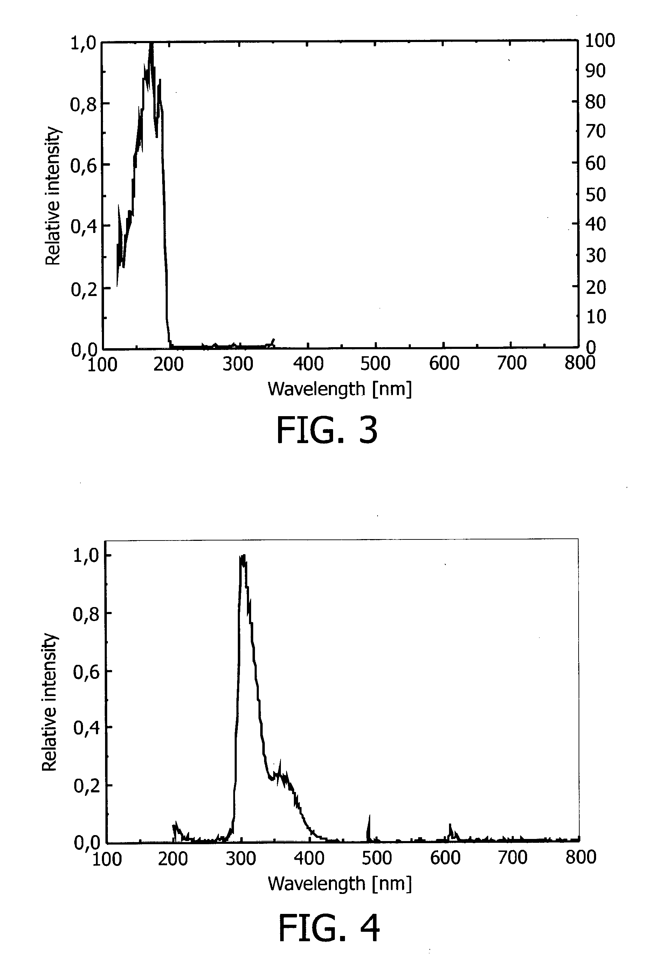

Fig. 2 shows the emission spectrum, Fig. 3 shows the excitation spectrum of the material of Example I. It can clearly be seen that this material is an excellent material for use in discharge lamps for UV-A radiation

### EXAMPLE II

Example II refers to Lu₃GaAl₄O₁₂:Pr, which was made in the following way:
The starting materials 59.093 g (148.5 mmol) Lu₂O₃, 19.577 g (192 mmol) Al₂O₃, 8.997 g (48 mmol) Ga₂O₃, and 3.064 g (3.0 mmol) Pr₆O₁₁ are dissolved in conc. HNO₃. Then the solvent is removed by evaporation and the remaining powder is fired for 2 h at 600 °C to decompose the nitrates.

Subsequently, the obtained is powderized and 1.680 g (20 mmol) AlF₃ are added as a flux. Afterwards, the powder is annealed for 2 h at 1300 °C in a CO atmosphere, powderized and fired again for 4 h between 1500 and 1700 °C in a CO atmosphere. Finally, the obtained powder cake is crushed and the powder is sieved through a 36 µm sieve.

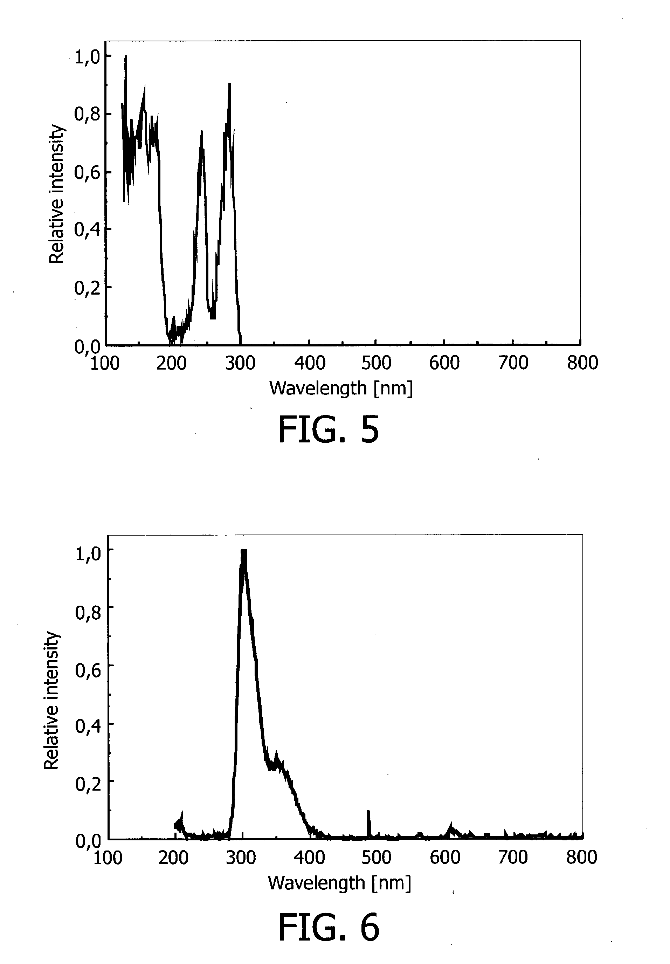

Fig. 4 shows the emission spectrum, Fig. 5 exhibits the excitation spectrum of the material of Example I. It is obvious that this material is an excellent material for use in discharge lamps for UV-B radiation.

### EXAMPLE III

Example III refers to Lu₃Ga₂Al₃O₁₂:Pr, which was made in analogous fashion to the material of Example II.

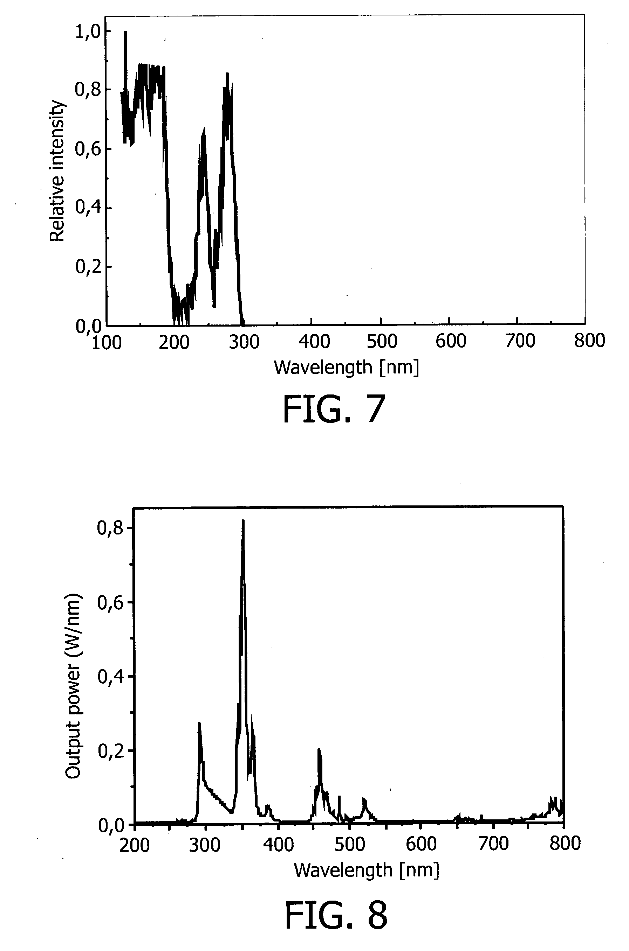

Fig. 6 shows the emission spectrum, Fig. 7 shows the excitation spectrum of the material of Example I. It can clearly be seen that this material is an excellent material for use in discharge lamps for UV-B radiation.

### EXEMPLARY XE EXCIMER DISCHARGE LAMP

The invention will furthermore be illustrated by means of an exemplary Xe excimer discharge lamp using the materials of Example I and II. This lamp was made in the following way:
A suspension of nanoparticles MgO is made on a butylacetate basis with nitrocellulose as a binder. The suspension is applied to the inner wall of a standard UV transparent glass tube by using a flow coat related procedure. Then, a suspension of a Lu₃GaAl₄O₁₂:Pr (Example II) and Lu₃Al₅O₁₂:Tm (Example I) is prepared on a butylacetate basis with nitrocellulose as a binder. By means of a similar flow coat related procedure, the suspension is applied to the inner wall of the precoated lamp tube with a typical phosphor layer weight in the range of 2 - 6 mg/cm². The binder is burned in a standard heating cycle with peak temperatures between 500 and 600 °C. The glass tube is filled with Xe using a thorough pumping cycle, so as to strictly exclude Oxygen impurities, and finally sealed. Typical gas pressures are 200 - 300 mbar pure Xe. Al-electrodes are attached to the outer side of the tube by means of adhesion or painting. The lamps are typically operated at 5 kV and 25 kHz, using a pulse driving scheme. The emission spectrum is determined using an optical spectrum multianalyser and is shown in Fig. 8.

## Claims

1. Discharge lamp, provided with a discharge vessel comprising a gas filling with a discharge-maintaining composition, wherein at least a part of a wall of the discharge vessel is provided with a luminescent material comprising (Lu_{1-x-y-a-b}YₓGd_{y})₃Al_{5z}Ga_{z}O₁₂:RE1ₐ:RE2_{b}, with RE1 being Tm³⁺ or mixtures thereof with Pr³⁺, RE2 being selected out of the group comprising Nd³⁺, Sar³⁺, Eu³⁺, Tb³⁺, Dy³⁺, Ho³⁺, Er³⁺ or mixtures thereof and a is >0 and ≤0.1, b is ≥0 and <0.1, x is ≥0 and ≤1, y is ≥0 and ≤1 (with the proviso that a+b+x+y are ≤1) and z is ≥0 and ≤5.

2. The discharge lamp of claim 1, wherein the discharge lamp is a Xe, Ne, or Xe/Ne excimer discharge lamp.

3. The discharge lamp of claim 1 or 2, wherein z is ≥0.5 and ≤5.

4. The discharge lamp of claim 3, wherein z is ≥1 and ≤3.

5. The discharge lamp of any one of the claims 1 to 4, wherein at least a part of a wall of the discharge vessel is provided with a basic metal oxide, preferably selected out of the group MgO, MgAl₂O₄, Al₂O₃, or Ln₂O₃ (Ln = Sc, Y, La, Gd, Lu) or mixtures thereof.

6. The discharge lamp of any one of the claims 1 to 5, wherein at least a part of a wall of the discharge vessel is provided with a further luminescent material selected out of the group comprising (Y₁₋ₓ₋ₐLuₓ)BO₃:Prₐ, (Y₁₋ₓ₋ₐLuₓ)PO₄:Prₐ, (Y₁₋ₓ₋ₐLuₓ)PO₄:Biₐ, La₁ₐPO₄:Prₐ, (Y₁₋ₓ₋ₐLuₓ)AlO₃:Prₐ, (Ca₁₋ₓ₋₂ₐSrₓ)₂P₂O₇:PrₐNaₐ, (Ca₁₋ₓ₋₂ₐSrₓ)Al₂O₁₉:CeₐNaₐ, or (La₁ₓ₋ₐGdₓ)PO₄:Ceₐ with x ≥0 and ≤1 and a is >0 and ≤0.1 (for all materials) or mixtures thereof.

7. Use of (Lu_{1-x-y-a-b}YGd_{y})₃Al_{5-z}Ga_{z}O₁₂:RE1ₐ:RE2_{b}, with RE1 being Tm³⁺ or mixtures thereof with Pr³⁺, RE2 being selected out of the group comprising Nd³⁺, Sm³⁺, Eu³⁺, Tb³⁺, Dy³⁺, Ho³⁺, Er³⁺ or mixtures thereof and a is >0 and ≤0.1, b is ≥0 and ≤0.1, x is ≥0 and ≤1, y is ≥0 and ≤1 (with the proviso that a+b+x+y are ≤1) and z is ≥0 and ≤5 as an activator in UV-A and/or UV-B emitting illumination systems.

8. A system comprising a discharge lamp according to any of the claims 1 to 6 and/or making use of claim 7, the system being used in one or more of the following applications:
Equipment for medical therapy (e.g. Treatment of skin diseases such as Psoriasis)
Equipment for cosmetic skin treatment (e.g. tanning devices)
Water sterilization and/or purification applications, e.g. by using an additional TiO₂ photocatalyst
Chemical reactors, e.g. for the photochemical synthesis of advanced chemical products, e.g. Vitamin D₃.

## Patentansprüche

1. Entladungslampe, versehen mit einem Entladungsgefäß, das eine Gasfüllung mit einer eine Entladung aufrechterhaltenden Zusammensetzung umfasst, wobei zumindest ein Teil einer Wandung des Entladungsgefäßes mit einem lumineszierenden Material versehen ist, enthaltend (Lui_{1-x-y-a-b}YₓGd_{y})₃Al_{5-z}Ga_{z}O₁₂:RE1ₐ:RE2_{b}, wobei es sich bei RE1 um Tm³⁺ oder Mischungen daraus mit Pr³⁺ handelt, RE2 aus der Gruppe umfassend Nd³⁺, Sm³⁺, Eu³⁺, Tb³⁺, Dy³⁺, Ho³⁺, Er³⁺ oder Mischungen daraus ausgewählt wird und a >0 und ≤0,1, b ≥0 und ≤0,1, x ≥0 und ≤1, y ≥0 und ≤1 ist (mit der Maßgabe, dass a+b+x+y≤1 sind) und z ≥ 0 und ≤5 ist.

2. Entladungslampe nach Anspruch 1, wobei die Entladungslampe eine Xe, Ne oder Xe/Ne-Excimer-Entladungslampe ist.

3. Entladungslampe nach Anspruch 1 oder 2, wobei z ≥0,5 und ≤5 ist.

4. Entladungslampe nach Anspruch 3, wobei z ≥1 und ≤3 ist.

5. Entladungslampe nach einem der Ansprüche 1 bis 4, wobei zumindest ein Teil einer Wandung des Entladungsgefäßes mit einem basischen Metalloxid versehen ist, das vorzugsweise aus der Gruppe MgO, MgAl₂O₄, Al₂O₃ oder Ln₂O₃ (Ln = Sc, Y, La, Gd, Lu) oder Mischungen daraus ausgewählt wird.

6. Entladungslampe nach einem der Ansprüche 1 bis 5, wobei zumindest ein Teil einer Wandung des Entladungsgefäßes mit einem weiteren lumineszierenden Material versehen ist, das aus der Gruppe ausgewählt wird, umfassend (Y₁₋ₓ₋ₐLuₓ)BO₃:Prₐ, (Y₁₋ₓₐLuₓ)PO₄:Prₐ, (Y₁₋ₓ-ₐLuₓ)PO₄:Biₐ, La₁₋ₐPO₄:Prₐ, (Y₁₋ₓ₋ₐLuₓ)AlO₃:Prₐ, (Ca₁₋ₓ₂ₐSrₓ)₂P₂O₇:PrₐNaₐ, (Ca₁₋ₓ₋₂ₐSrₓ)Al₁₂O₁₉:CeₐNaₐ, oder (La₁₋ₓ₋ₐGdₓ)PO₄:Ceₐ, wobei x ≥0 und ≤1 und a >0 und ≤0,1 ist (für alle Materialien oder Mischungen daraus).

7. Verwendung von (Lu_{1-x-y-a-b}YₓGdy)₃Al_{5-z}Ga_{z}O₁₂:RE1ₐ:RE2_{b}, wobei es sich bei RE1 um Tm³⁺ oder Mischungen daraus mit Pr³⁺ handelt, RE2 aus der Gruppe umfassend Nd³⁺, Sm³⁺, Eu³⁺, Tb³⁺, Dy³⁺, Ho³⁺, Er³⁺ oder Mischungen daraus ausgewählt wird und a >0 und ≤0,1, b ≥0 und ≤0,1, x ≥0 und ≤1, y ≥0 und ≤1 ist (mit der Maßgabe, dass a+b+x+y≤1 sind) und z ≥ 0 und ≤5 ist, als ein Aktivator in UV-A und/oder UV-B emittierenden Beleuchtungssystemen.

8. System mit einer Entladungslampe nach einem der Ansprüche 1 bis 6 und/oder unter Verwendung von Anspruch 7, wobei das System für eine oder mehrere der folgenden Zwecke eingesetzt wird;
Geräte zur medizinischen Therapie (z.B. Behandlung von Hautkrankheiten, wie z.B. Psoriasis)
Geräte zur kosmetischen Hautbehandlung (z.B. Bräunungsgeräte)
Wassersterilisierungs- und/oder Reinigungsanwendungen, z.B. unter Verwendung eines zusätzlichen TiO₂ Photokatalysators
Chemische Reaktionsapparate, z.B. für die photochemische Synthese hoch entwickelter chemischer Produkte, z.B. Vitamin D₃.

## Revendications

1. Lampe à décharge, dotée d'une enceinte de décharge comprenant un remplissage en gaz avec une composition de maintien de décharge, dans laquelle au moins une partie d'une paroi de l'enceinte de décharge est dotée d'une substance luminescente comprenant du (Lu_{1-x-y-a-b}YₓGd_{y})₃Al_{5-z}Ga_{z}O₁₂:RE1ₐ:RE2_{b}, RE1 étant du Tm³⁺ ou des mélanges de celui-ci avec du Pr³⁺, RE2 étant choisi dans le groupe comprenant Nd³⁺, Sm³⁺, Eu³⁺, Tb³⁺, Dy³⁺, Ho³⁺, Er³⁺ ou des mélanges de ceux-ci et a étant > 0 et ≤ 0,1, b étant ≥ 0 et ≤ 0,1, x étant ≥ 0 et ≤ 1, y étant ≥ 0 et ≤ 1 (à condition que a + b + x + y soient ≤ 1) et z étant ≥ 0 et ≤ 5.

2. Lampe à décharge selon la revendication 1, la lampe à décharge étant une lampe à décharge à excimère Xe, Ne ou Xe/Ne.

3. Lampe à décharge selon la revendication 1 ou 2, dans laquelle z est ≥ 0,5 et ≤ 5.

4. Lampe à décharge selon la revendication 3, dans laquelle z est ≥ 1 et ≤ 3.

5. Lampe à décharge selon l'une quelconque des revendications 1 à 4, dans laquelle au moins une partie d'une paroi de l'enceinte de décharge est dotée d'un oxyde de métal de base, de préférence choisi dans le groupe constitué de MgO, MgAl₂O₄, Al₂O₃ ou Ln₂O₃ (Ln = Sc, Y, La, Gd, Lu) ou de mélanges de ceux-ci.

6. Lampe à décharge selon l'une quelconque des revendications 1 à 5, dans laquelle au moins une partie d'une paroi de l'enceinte de décharge est dotée d'une autre substance luminescente choisie dans le groupe comprenant (Y₁₋ₓ₋ₐLuₓ)BO₃:Prₐ, (Y₁₋ₓₐLuₓ)PO₄:Prₐ, (Y₁₋ₓ₋ₐLuₓ)PO₄:Biₐ, La₁₋ₐPO₄:Prₐ, (Y₁₋ₓ-ₐLuₓ)AlO₃:Prₐ, (CA₁₋ₓ₋₂ₐSrₓ)₂P₂O₇:PrₐNaₐ, (CA₁₋ₓ₋₂ₐSrₓ)Al₁₂O₁₉:CeₐNaₐ ou (La₁₋ₓ₋ₐGdₓ)PO₄:Ceₐ, x étant ≥ 0 et ≤ 1 et a étant > 0 et ≤ 0,1 (pour toutes les substances) ou des mélanges de celles-ci.

7. Utilisation de (Lu_{1-x-y-a-b}YₓGd_{y})₃Al_{5-z}GaO₁₂:RE1ₐ:RE2_{b}, RE1 étant du Tm³⁺ ou des mélanges de celui-ci avec du Pr³⁺, RE2 étant choisi dans le groupe comprenant Nd³⁺, Sm³⁺, Eu³⁺, Tb³⁺, Dy³⁺, Ho³⁺, Er³⁺ ou des mélanges de ceux-ci et a étant > 0 et ≤ 0,1, b étant ≥ 0 et ≤ 0,1, x étant ≥ 0 et ≤ 1, y étant ≥ 0 et ≤ 1 (à condition que a + b + x + y soient < 1) et z étant ≥ 0 et ≤ 5 en tant qu'activateur dans des systèmes d'éclairage émettant des UV-A et/ou des UV-B.

8. Système comprenant une lampe à décharge selon l'une quelconque des revendications 1 à 6 et/ou ayant une utilisation selon la revendication 7, le système étant utilisé dans une ou plusieurs des applications suivantes :
un équipement de thérapie médicale (par exemple, de traitement des maladies de la peau telles que le psoriasis)
un équipement de traitement esthétique de la peau (par exemple, des dispositifs de bronzage)
des applications de stérilisation et/ou d'épuration de l'eau, par exemple, au moyen d'un photocatalyseur TiO₂ supplémentaire
des réacteurs chimiques, par exemple, pour la synthèse photochimique de produits chimiques élaborés, par exemple de la vitamine D₃.
